# EUROPEAN PATENT APPLICATION

(11) **EP 4 201 417 A1**
(43) Date of publication of application: **28.06.2023**
(21) Application number: 20955366.8
(22) Date of filing: 22.10.2020
(51) Int. Cl.: A61K 35/747, A61P 25/28, A23L 33/135, A61K 8/99, A61Q 19/00, A23K 10/18

(54) **COMPOSITION FOR PREVENTION OR TREATMENT OF ALZHEIMER'S DISEASE COMPRISING LACTOBACILLUS SAKEI PROBIO-65**

(30) Priority: 23.09.2020 KR 20200122716
(71) Applicant: Probionic Inc., Jeonju-si, Jeollabuk-do 54810 (KR)
(72) Inventor: PARK, Yong-Ha, Seoul 03165 (KR); LIONG, Min-Tze, Penang, 11900 (MY)
(74) Representative: Isarpatent
(86) International application number: PCT/KR2020/014460
(87) International publication number: WO 2022/065568

(57) **Abstract**

The present invention relates to a composition for prevention or treatment of Alzheimer's disease comprising *Lactobacillus sakei* Probio-65, and more particularly, it relates to a composition for the prevention or treatment of Alzheimer's disease containing *Lactobacillus sakei* Probio-65 which may alleviate Alzheimer's disease through changes in the profile of intestinal microorganisms and intestinal metabolome composition without side effects.

## Description

### BACKGROUND OF THE INVENTION

### 1. Field

The present invention relates to a composition for prevention or treatment of Alzheimer's disease comprising *Lactobacillus sakei* Probio-65, and more particularly, it relates to a composition for the prevention or treatment of Alzheimer's disease containing *Lactobacillus sakei* Probio-65 which may alleviate Alzheimer's disease through changes in the profile of intestinal microorganisms and intestinal metabolome composition without side effects.

### 2. Description of the Related Art

*Lactobacillus sakei* Probio-65 is a strain isolated from kimchi, a fermented food which has been consumed by Koreans for hundreds of years, and of which safety has been traditionally secured. It exhibits acid resistance, bile resistance, and antibiotic resistance, suppresses the growth of harmful pathogenic microorganisms in the body and intestines of animals, and exhibits immunity enhancing activity. In particular, it is effective in treating or preventing atopic dermatitis by suppressing the growth of Staphylococcus aureus, which is known as a disease exacerbation factor of atopic dermatitis lesions. In addition, it is effective in treating or preventing allergy-related diseases, and has an effect of stabilizing the intestinal microbial flora by inhibiting the abnormal growth of harmful microorganisms in the intestine. The inventors of Korean Patent Registration No. 10-0479719 have proposed the *Lactobacillus sakei* Probio-65 and its composition.

The present inventors found that *Lactobacillus sakei* Probio-65 may be used as a composition for preventing or treating Alzheimer's disease while conducting research on other efficacies of *Lactobacillus sakei* Probio-65.

Alzheimer's disease is the most prevalent dementia worldwide, affecting 47 million people worldwide and costing a total of $604 billion as of 2018(Alzheimer's Association, 2018). Alzheimer's disease shares many pathological aspects with other neurodegenerative diseases, such as tangles of nerve fibers and loss of neurons, primarily in the cerebral cortex and hippocampus. However, a hallmark of this disease is the presence of amyloid plaques caused through the deposition of an amyloidogenic peptide known as amyloid beta(Aβ). Among all the Aβ species formed, Aβ42 is the most toxic form and the aggressive one causing neurodegeneration. Inflammation is a central mechanism in Alzheimer's disease where the homeostasis of the inflammatory response is disrupted, leading to chronic inflammation and early Aβ pathology.

Specifically, Aβ, which is currently known as the most likely cause of Alzheimer's disease, is produced when beta(β) and gamma secretase(y-secretase) enzymes abnormally hydrolyze APP(Amyloid Precursor Protein). It is well known that Aβ contains toxicity and destroys the nerve cells of the cerebral cortex and cerebral cortex which produce acetylcholine and a neurotransmitter, thereby reducing the active state of acetylcholine, and generating reactive oxygen species(ROS), and causing neuronal cell death by limiting oxidative damage.

Most of the currently used Alzheimer's disease therapeutics are Ach lyase inhibitors, but have problems in that they have a large side effect of liver toxicity and cause various side effects.

In order to solve these problems of conventional Alzheimer's disease therapeutics, the present inventors have developed a composition for preventing or treating Alzheimer's disease. The composition has no side effects in the body and includes *Lactobacillus sakei* Probio-65, which may alleviate Alzheimer's disease through changes in the intestinal microbial profile and intestinal metabolome composition.

### SUMMARY OF THE INVENTION

The technical object to be achieved by the present invention is to provide a composition for prevention or treatment of Alzheimer's disease and the composition includes *Lactobacillus sakei* Probio-65 which has no side effects in the body, may alleviate Alzheimer's disease through changes in the intestinal microbial profile and intestinal metabolome composition, and may be used in pharmaceutical compositions, food compositions, cosmetics and feed compositions based on microorganisms or their cultures.

The above and other objects of the present invention may all be achieved by the present invention described below.

A composition for prevention or treatment of Alzheimer's disease comprising *Lactobacillus sakei* Probio-65 according to an embodiment of the present invention is characterized in that it contains *Lactobacillus sakei* Probio-65 as an active ingredient.

Here, the *Lactobacillus sakei* Probio-65 is characterized in that it is a *Lactobacillus sakei* Probio-65 culture medium obtained by liquid culture of the *Lactobacillus sakei* Probio-65 strain.

In addition, the composition for preventing or treating Alzheimer's disease containing *Lactobacillus sakei* Probio-65 according to an embodiment of the present invention may be used as a food composition containing *Lactobacillus sakei* Probio-65 or a culture medium thereof.

A composition for preventing or treating Alzheimer's disease containing *Lactobacillus sakei* Probio-65 according to an embodiment of the present invention may be used as a cosmetic containing *Lactobacillus sakei* Probio-65 or its culture medium.

A composition for prevention or treatment Alzheimer's disease comprising Lactobacillus Sake Probio-65 according to an embodiment of the present invention may be used as a feed composition containing *Lactobacillus sakei* Probio-65 or a culture medium thereof.

The present invention has an effect that there is provided a composition for preventing or treating Alzheimer's disease, and including *Lactobacillus sakei* Probio-65, which has no side effects in the body and may alleviate Alzheimer's disease through changes in the profile of intestinal microorganisms and intestinal metabolome composition.

### BRIEF DESCRIPTION OF THE DRAWINGS

FIG. 1 is a photograph taken at 800 times magnification of the eye shape of GMR-OreR supplied with normal food.
FIG. 2 is a photograph taken at 800 times magnification of the eye morphology of transgenic fruit flies(GMR-Aβ42) supplied with normal food.
FIG. 3 is a photograph taken at 800 times magnification of the eye morphology of transgenic Drosophila (MR-Aβ42) supplied with normal food containing the *Lactobacillus sakei* Probio-65 culture medium.
FIG. 4 is a graph showing the principal coordinate analysis (PcoA) of the microbial community based on the Bray-Curtis distance matrix.
FIG. 5 is a graph showing the intestinal microbiological characteristics of Drosophila showing the relative abundance in the genus observed in different treatment groups.

### DETAILED DESCRIPTION OF THE INVENTION

The above-described objects, features and advantages of the present invention will become more apparent by explaining in detail preferred embodiments and comparative examples of the present invention. The detailed description which will be described later is not to be described in a limiting sense, and the scope of the present invention is limited only by the appended claims, along with all equivalents as claimed by those claims.

Hereinafter, the present invention will be described in more detail with reference to the accompanying drawings and embodiments.

### Embodiment

### Embodiment 1: Preparation of culture medium of Lactobacillus sakei Probio-65

*Lactobacillus sakei* Probio-65, isolated from kimchi, a traditional Korean fermented food, was put into an incubator and subcultured 2-3 times at 37°C for 15 hours in MRS broth (Difco laboratories, USA) medium to improve the physiological activity of long-term preserved strains and to select healthy strain colony. The activated culture were cultured for 15 hours while being maintained at a constant temperature in an incubator at 37 °C by using MRS broth medium, and was used as a seed inoculum. Then, 1% (w/v) of glucose, 1% (w/v) of yeast extract, 0.25% (w/v) of soy peptone, 0.1% (w/v) of polyoxyethylene sorbitan mono-oleate, 0.1% (w/v) of ammonium sulfate ((NH₄)₂SO₄), 0.01% (w/v) of magnesium sulfate (MgSO₄), 0.1% (w/v) of potassium dihydrogen phosphate (KH₂PO₄) and 90 to 99 weight% (w/v) of water was sterilized at 121 °C for 15 minutes. Thereafter, the *Lactobacillus sakei* Probio-65 culture medium was prepared by liquid-cultivating the *Lactobacillus sakei* Probio65 in a 2L flask at 37° C for 24 hours using this medium.

### Embodiment 2: Alzheimer's disease prevention or treatment effect test of Lactobacillus sakei Probio-65 using transgenic Drosophila (GMR-AP42)

### Embodiment 2-1 Breeding of Experimental Drosophila Groups

Drosophila melanogaster for this experiment were Oregon-R wild species; Glass Multiple Reporter (GMR)-GAL4; and UAS-Aβ42 were used. Oregon-R was crossed with GMR-GAL4 to produce GMR-OreR, an UAS-Aβ42 was crossed with GMR-GAL4 to produce transgenic Drosophila (GMR-Aβ42). All Drosophila groups were grown in an environment of 25 °C, and mating conditions were maintained at 30 °C.

### Embodiment 2-2 Food supply to experimental Drosophila groups

Before transformation treatment, Drosophila groups for all experiments were grown in an environment of 18°C and supplied with normal food. As a general feed, boiled corn starch 4% (w/v), polenta 5% (w/v), brown sugar 10%(w/v), agar 0.7% (w/v), heat-killed yeast 5% (w/v), Nipagin 3% (w/v) and Propionic acid 0.7% (v/v) were mixed, and then transferred to a sterile plastic vial, followed by cooling and solidification. In the transformation process, the same diet was prepared without nipagin and propionic acid, *and Lactobacillus sakei* Probio-65 (1×10¹¹ CFU/ mf) was added just before solidification in the cooling process of the diet. Each feed was prepared fresh before feeding. Normal and transgenic Drosophila (GMR-Aβ42) were raised on normal diet. On the other hand, the transgenic Drosophila (GMR-Aβ42) fed with *Lactobacillus sakei* Probio-65 grew upon a diet containing lactic acid bacteria. Offspring Drosophila were collected after 10 days from each group for analysis.

### Experimental results of Embodiment 2-3

As Drosophila shares a similar but simpler central nervous system with mammals, the use of Drosophila melanogaster among Drosophila in neurological research represents a major breakthrough in this field. Therefore, in this experiment, Drosophila melanogaster (D. melanogaster) was used as the group of fruit flies. In addition, the culture composition containing *Lactobacillus sakei* Probio-65 according to the present invention was tested on D. melanogaster ectopically expressing human Aβ42 in compound eyes using a pan-retinal GMR-GAL4 driver.

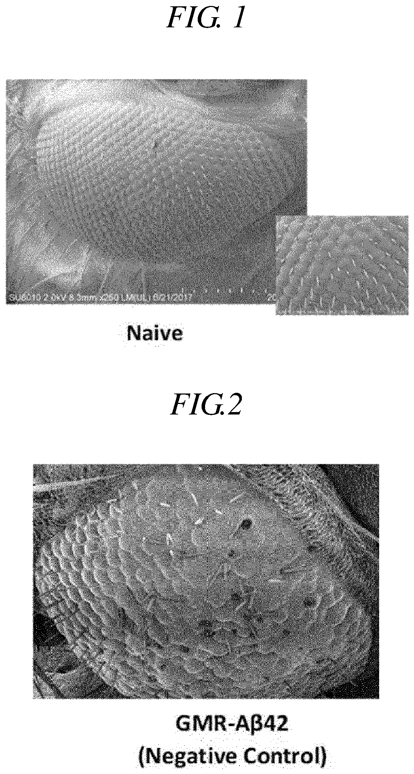

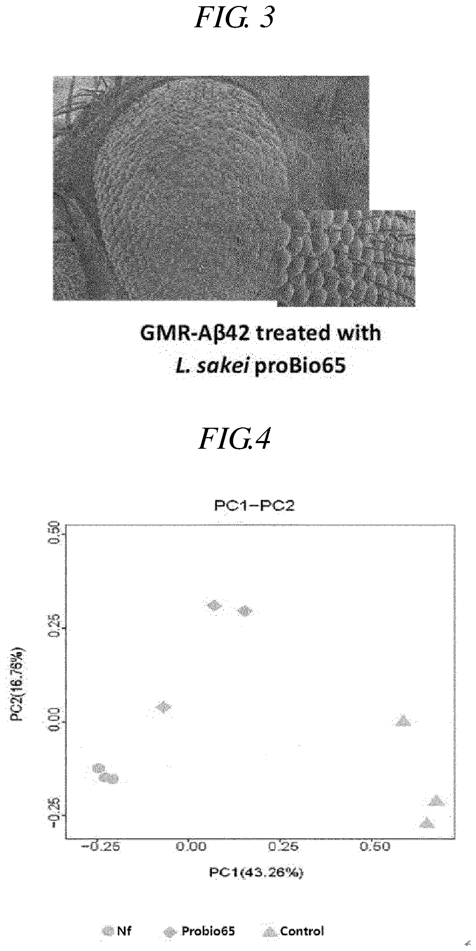

FIG. 1 is a photograph of the eye shape of GMR-OreR supplied with normal food at 800 times magnification, and IG. 2 is a photograph of the eye shape of transgenic fruit fly (GMR-Aβ42) supplied with normal food at 800 times magnification. FIG. 3 is a photograph of the eye shape of transgenic fruit fly (GMR-Aβ42) supplied with normal food containing *Lactobacillus sakei* Probio-65 culture medium at 800 times magnification.

As may be seen in FIGS. 1 to 3, in the eye shape of GMR-OreR (control group) supplied with normal food, small hexagonal shapes are regularly arranged and bristles are formed between the hexagonal shapes. However, transgenic Drosophila (GMR-Aβ42) fed with normal diet (negative control) showed a strong neurodegenerative phenotype characterized by a rough eye phenotype (REP). As a result of comparing this test to the negative control group, in connection with transgenic Drosophila (GMR-Aβ42) fed normal food containing *Lactobacillus sakei* Probio-65 (1×10¹¹ CFU/mℓ), less visible omatidia together with each unit having a small hexagonal shape was observed, and a large increase in the number of setae and a rectification of the angle of the setae were appeared.

**[Table 1] Degree of structure effect on rough eye phenotype**

| **Strain** | **degree of structure effect** | | |
|---|---|---|---|
| | **Hexagon compound eye** | **Number of bristles** | **Presence or absence of hole** |
| GMR-Aβ42 without treatment | - | - | - |
| GMR-A42 treated with L. *sakei* ProBio65 | ++ | + | ++ |
| Control | +++ | +++ | +++ |

Table 1 is a table showing the degree of structural effect on the rough eye phenotype with respect to FIGS. 1 to 3. As may be seen in Table 1, it was shown that as the neurotoxin effect was reduced due to the supply of *Lactobacillus sakei* Probio-65, Aβ42 decreased. The step of the reduction effect was very conspicuous in the holes of hexagonal compound eyes and single eyes (Omatidia).

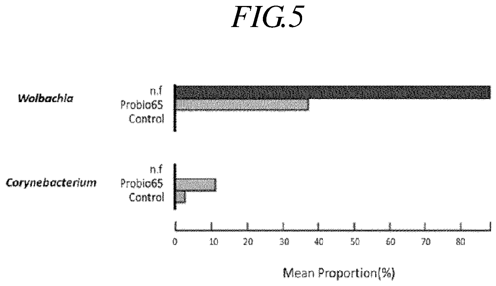

FIG. 4 is a graph showing the principal coordinate analysis (PcoA) of the microbial community based on the Bray-Curtis distance matrix, and FIG. 5 is a graph showing the intestinal microbiological characteristics of Drosophila showing the relative abundance in the genus observed in different treatment groups. In FIG. 4 and FIG. 5, Nf is the transgenic Drosophila (GMR-Aβ42)(negative control) which is provided with normal food, Probio65 is transgenic Drosophila (GMR-Aβ42) (Probio65 treatment group) which is provided with normal food containing *Lactobacillus sakei* Probio-65 (1×10 ¹¹ CFU/mt), and Control represents GMR-OreR (control) which is provided with normal food.

As may be seen in Figure 4, as a result of the analysis of the intestinal microflora, the Probio65 treatment group was found to be more similar to the control group than the negative control group. In addition, as may be seen in FIG. 5, after treating transgenic Drosophila with *Lactobacillus sakei* Probio-65, the relative abundance of Wolbachia, a type of parasitic bacteria, was decreased by 37.9% in the Probio65-treated group, whereas Corynebacterium_1 showed a high level only in the Probio65 treated group. Wolbachia is reported as a potential biomarker for Alzheimer's disease because it is the initiator of Alzheimer's disease or a dominant bacterium in the pathogenesis in D.melanogaster. Based on preliminary studies of patients with Alzheimer's disease, it has been reported that Alzheimer's patients have low plasma D-glutamate levels. Also, reduced D-glutamate levels in the brain are associated with cognitive impairment in Alzheimer's disease. These data highlight the importance of D-glutamate in the mental health of patients with Alzheimer's disease. In addition, *Lactobacillus sakei* Probio-65 enhances the growth of Corynebacterium present in the intestines of transgenic Drosophila, thereby potentially promoting glutamate production. Thus, the transgenic Drosophila showed significant Alzheimer's disease alleviating effects. From the above experimental results, it was confirmed that *Lactobacillus sakei* Probio-65 has an effect that neurodegeneration expressed in eye defects of Drosophila is improved through changes in the intestinal microbial profile and intestinal metabolome composition.

Although preferred embodiments of the present invention have been described above, the present invention is not limited to the specific embodiments described above. That is, those skilled in the art to which the present invention belongs may make many changes and modifications to the present invention without departing from the spirit and scope of the appended claims, and all such appropriate changes, modifications, and the equivalents should also be considered as falling within the scope of this invention.

The composition containing *Lactobacillus sakei* Probio-65 of the present invention has a useful for alleviating Alzheimer's disease, and may be used in various pharmaceutical compositions, food compositions, cosmetic and feed compositions in the form of microorganisms or their culture medium.

## Claims

1. A composition for prevention or treatment of Alzheimer's disease comprising *Lactobacillus sakei* Probio-65 as an active ingredient.

2. The composition for prevention or treatment of Alzheimer's disease according to claim 1, wherein the *Lactobacillus sakei* Probio-65 is a *Lactobacillus sakei* Probio-65 culture medium obtained by liquid-cultivating the *Lactobacillus sakei* Probio-65 strain.

3. A food composition comprising *Lactobacillus sakei* Probio-65 or a culture medium thereof.

4. Cosmetics containing *Lactobacillus sakei* Probio-65 or its culture medium.

5. A feed composition comprising *Lactobacillus sakei* Probio-65 or a culture medium thereof.
